# EUROPEAN PATENT SPECIFICATION

(11) **EP 4 398 199 B1**
(45) Date of publication and mention of the grant of the patent: **29.07.2026**
(21) Application number: 23150196.6
(22) Date of filing: 03.01.2023
(51) Int. Cl.: G06V 10/25, G06V 10/44, G06V 10/764, G06V 10/82, G06V 10/84, G06V 10/94, G06N 3/0464, G06N 3/09

(54) **METHOD OF CLASSIFYING AN IMAGE FEATURE OF A CARDIAC IMAGE IN AN ELECTRONIC IMAGE PROCESSING SYSTEM, AND SYSTEM THEREFOR**
VERFAHREN ZUR KLASSIFIZIERUNG EINES BILDMERKMALS EINES HERZBILDES IN EINEM ELEKTRONISCHEN BILDVERARBEITUNGSSYSTEM UND SYSTEM DAFÜR
PROCÉDÉ DE CLASSIFICATION D'UNE CARACTÉRISTIQUE D'IMAGE D'UNE IMAGE CARDIAQUE DANS UN SYSTÈME DE TRAITEMENT D'IMAGE ÉLECTRONIQUE, ET SYSTÈME ASSOCIÉ

(43) Date of publication of application: 10.07.2024
(73) Proprietor: FEops NV, 9052 Gent (BE)
(72) Inventor: NAUWYNCK, Maxime, B-9052 Gent (BE); HEFFINCK, Eva, B-9052 Gent (BE); MICHIELS, Kilian, B-9052 Gent (BE)
(74) Representative: V.O.

(56) References cited:
- US-A1- 2021 264 589
- SCHOBS LAWRENCE ET AL: "Confidence-Quantifying Landmark Localisation For Cardiac MRI", 2021 IEEE 18TH INTERNATIONAL SYMPOSIUM ON BIOMEDICAL IMAGING (ISBI), IEEE, 13 April 2021 (2021-04-13), pages 985 - 988, XP033918162, [retrieved on 20210517], DOI: 10.1109/ISBI48211.2021.9433895

## Description

### Field of the invention

The present invention is directed at a method of classifying an image feature in an electronic image processing system, in order to determine, from cardiac image data, a cardiac characteristic of a patient by using a trained machine learning data processing model, wherein the trained machine learning data processing model is trained to evaluate a probability that the image feature matches each class of a predetermined plurality of classes forming a set of classes such as to characterize the image feature, the cardiac image data comprising at least a region of interest including the image feature, wherein the method comprises: receiving, by the trained machine learning data processing model, the cardiac image data; obtaining, by the trained machine learning data processing model, data indicative of the region of interest; collecting, from the trained machine learning data processing model, probability data, the probability data comprising for each class of the set of classes a probability that the image feature matches the respective class. The invention is further directed at a method of training a machine learning data processing model for performing a method as described, and at an electronic image processing system.

### Background

The current state of technology in medical imaging provides many different imaging techniques that together allow for detection and evaluation of many different characteristics or conditions, in an ever early stage. However, regardless of this, in many cases the proper evaluation still remains challenging. This may be for a large variety of reasons, for example: the imaged subject is in continuous motion, the obtainable resolution is not sufficient to reveal all necessary details, the size and shape of a subject renders it difficult to resolve or identify on image, the image available is two-dimensional and renders it difficult to do the evaluation, the differences in tissue structure provide a low contrast, etcetera.

An example wherein the evaluation of some health conditions is difficult to perform is in the field of cardiac imaging. The continuous motion of a living heart combined with the material parameters of the tissue, diminishes the image quality such that various characteristics or conditions cannot be detected with sufficient certainty from a cardiac image. In certain doubtful cases, a same image being evaluated by multiple physicians may not lead to a decisive conclusion due to disagreement between the physicians being fed by uncertainty on what an image really shows. This of course is not desired. Although image recognition based on artificial intelligence has improved this, it has not yet completely resolved the issue. The method disclosed by 'Lawrence Schobs et al., Confidence-Quantifying Landmark Localisation For Cardiac MRI', is a quantifying model for landmark localisation for cardiac MRI. It uses a candidate smoothing strategy to fuse multi-task outputs to generate the final prediction and quantify the confidence. Although quantifiying the confidence is a first step in optimising image recognition techniques, physicians still have to deal with uncertainty of analysis accuracy as there are many factors that can influence the interpretation of image detection analysis results.

### Summary of the invention

It is an object of the present invention to ameliorate the evaluation of cardiac images, and provide a method and system for classifying an image feature thereof, in particular to facilitate decision making by a physician.

To this end, there is provided herewith in accordance with a first aspect of the invention, a method as described above, wherein the method further comprises, the steps of: evaluating, by a controller, the probability data for the classes of the set of classes, and identifying one or more candidate classes having a significant probability of matching with the image feature; determining a certainty level on the basis of the probability data for each of the identified candidate classes based on a comparison of the probabilities obtained in the evaluation step, wherein the certainty level is indicative of the certainty that the image feature matches a particular candidate class; and dependent on the step of determining, performing at least one step of: when the number of candidate classes equals one, and the certainty level exceeds a threshold level: associating the one candidate class with the image feature; or when the number of candidate classes equals one, and the certainty level is smaller than a threshold level: requesting user input indicative of a confirmation or disaffirmation that the one candidate class matches with the image feature; or when the number of candidate classes exceeds one: requesting, for each of the one or more candidate classes, user input indicative of a confirmation or disaffirmation that the respective candidate class matches with the image feature.

The method of the present invention classifies image features, in cardiac image data, to match (or not match) a cardiac characteristic of a patient, and uses therefore a trained machine learning data processing model. In particular, the machine learning data processing model evaluates the probability of each class of a predetermined plurality of classes forming a set of classes in order to characterize the image feature. Thus, a closed set of predetermined classes is to be evaluated as to whether or not it is likely that they occur. The closed set will be mutually exclusive for the condition, i.e. if one of the classes applies, none of the other classes apply. In accordance with the present invention, the trained machine learning data processing model provides probability data, which for each class of the set of classes indicates a probability that the image feature matches the respective class. The probability data may for example provide a probability distribution which describes this probability, or alternatively the outcome thereof for the particular case. This class probability data may then be evaluated by the controller for each of the classes of the set of classes. One or more candidate classes may therefrom be identified, that have a significant probability of matching with the image feature. For example, a significant probability may be any of: an above average or mean probability as compared to probabilities of other (candidate) classes, a maximum probability or the top number of highest of probabilities (e.g. top three highest) of the various probabilities associated with each of a plurality of classes, a probability being significantly higher than the nearest probability below, or the 5^{th}, 10^{th}, 15^{th} or n^{th} percentile (0 < n < 50) of largest probabilities. Based on a comparison between the probabilities obtained in the evaluation step, a certainty level is determined or estimated on the basis of the probability data for each of the identified candidate classes, wherein the certainty level is indicative of the certainty that the image feature matches a particular candidate class. The final outcome of the method will be dependent on the certainty level, and in accordance with the method is determined as follows. In this connection, the term 'certainty level' must be interpreted sufficiently broad to include a numeric or real value (e.g. a percentage or fraction), or a certainty class (e.g. 'low', 'medium', 'high'; or 0%-10%, 10%-20%, 20%-30%, ...., 90%-100%; or 'very unlikely', 'unlikely', 'average', 'likely', 'very likely').

Dependent on the step of determining the certainty level, in accordance with the method, when in the step of identifying candidate classes only a single candidate class remains (i.e. the number of candidate classes equals one) and the certainty level exceeds a threshold level, the one candidate class is associated with the image feature. The threshold level in that case should not leave any room for a different outcome. The threshold level may be dependent on various factors, and may be predetermined as a setting in the system. For example, the threshold level may be set to 70%, 75%, 80%, 85%, 90% or 95% (e.g. anything between 65% and 100%) dependent on preference. Furthermore, the threshold level does not have to be constant, but can be a non-linear function of the descriptive statistics of the probability distribution, e.g. determined by the controller or by another entity.

When, however, in accordance with the method the number of candidate classes equals one and the certainty level is smaller than a threshold level, the method may provide this as output to the user of the system and request input from the user. This input may be indicative of a confirmation or disaffirmation that the user (typically a physician) considers the one candidate class to match with the image feature. In certain embodiments, in accordance with the method the system may provide the probabilities or certainty levels to the user and/or may indicate which class is considered to provide the most likely outcome.

When, in accordance with the method, more than one candidate class may be possible (i.e. the number of candidate classes exceeds one), the method may continue with indicating this to the user and request user input. In this case, user input indicative of a confirmation or disaffirmation that the respective candidate class matches with the image feature is requested to the user.

The present invention improves the decision making process. Due to the fact that all classes of the closed set of mutually exclusive classes are evaluated against their probabilities of occurring based on the image data, a proper outcome may in more situation be decisively provided by the system. This diminishes the chance on an incorrect diagnosis. At the same time, the system does not steer the user in a wrong direction, because in those cases that are not completely certain this is flagged to the user and user input is requested. The system thus only improves the current situation, while preventing an incorrect conclusion or diagnose based on an (uncertain) appearance of a characteristic or condition. This is because the system is better able to judge whether it is indeed challenging or impossible to make a good judgement.

In some embodiments, the class probability data may be expanded with counter-class probability data, i.e. probability data that is indicative of a probability that the image feature does not match the respective class. Normally, the sum of class probability data and counter-class probability data will add up to 100% (this may optionally even be assumed), although this may not be per definition the case, as will be explained further below. The counter-class probability data may be used in the determination of the certainty level. If for example one candidate class appears to be most likely, then for each of the other classes the counter-class probability data must indicate to be most likely for that particular class (i.e. indicative of the class being not likely).

In many cases, the class probability data and the counter-class probability data may add up to 100%. However, in some cases, imperfections in the image (i.e. noise, disturbances, artefacts, etc.) may result in the sum of the class and counter-class probability data not to add up to 100%. The difference between this sum and 100%, in that case, may be indicative of image quality with respect to enabling evaluation of the respective class or may be indicative of the existence of one or more additional unknown classes. In images with insufficient image quality, wherein no clear and decisive probability on whether or not the image features matches the class can be provided, the method may signal this outcome to a user and also indicate that this is due to an insufficient quality of an image. This prevents the establishment of an incorrect conclusion or diagnose where a picture is indecisive.

In other or further embodiments, the method further comprises providing output to a user of the system, wherein the output comprises at least one element of a group comprising: data indicative of the one or more candidate classes; data of an association between one candidate class and the image feature; the certainty level; or the probability data. Typically, in accordance with the method, an association between a certain class and an image feature may be provided.

For example, considering a case of determining aortic valve morphology, the set of classes evaluated may include any one or more of: bicuspid aortic valve type 0 (BAV-0), bicuspid aortic valve type 1 (BAV-1), tricuspid aortic valve (TAV), quadricuspid aortic valve (QAV), pentacuspid aortic valve (PAV), unicuspid aortic valve (UAV) or n-cuspid aortic valve. Typically, the first three of these (BAV-0, BAV-1 and TAV) more frequently occur. The trained machine learning data processing model may be trained to consider the above mutually exclusive classes, and determine from a cardiac image at the input, whether any of the image features in the region of interest gives rise to an association with any of the above classes. Probability data indicative on the occurrence of each of these classes may be obtained from the parameters of the trained machine learning data processing model. Optionally, also probability data of counter-classes may be determined. The counter-classes are complementary to each class, e.g. for the class BAV-0 the counter-class is NOT_BAV-0 (the probability that the image feature is indicative of not BAV-0), for the class BAV-1 the counter-class is NOT_BAV-1 and for the class TAV the counter-class is NOT_TAV, etc. Once an image feature in accordance with the method can be associated with a class (or a counter-class), this association may be presented as output of the system to the user. Also, optionally the certainty level for this may be presented as output, and if desired also the probability data for each class (and/or counter-class) may be presented as output.

In some embodiments, for providing the probability data, the machine learning data processing model comprises at least one Gaussian layer in order to determine for each class of the set of classes said probability that the image feature matches the respective class. For example, the use of a Gaussian layer in an artificial neural network directly enables to obtain probability data for a certain outcome. This may be obtained for each class (and optionally each counter-class). Alternatively, similar results may be obtained by applying a Bayesian network, which provides a good alternative for obtaining probability data.

In some embodiments, the trained machine learning data processing model is a convolutional neural network including one or more intermediate layers, and wherein the step of collecting further comprises: obtaining from at least one of the intermediate layers, a weighting vector of M weights and intermediate data of M channels, wherein M comprises a natural number larger than zero; weighting the intermediate data using the weights resulting in weighted intermediate data; and sampling the weighted intermediate data resulting in region-indicating data, the region-indicating data being indicative of one or more regions of the cardiac image having an above average weighing in the weighted intermediate data.

In other or further embodiments, the trained machine learning data processing model comprises an input layer for receiving at least the cardiac image data, and an output layer for providing output data, the output data including at least one of: data indicative of the one or more candidate classes, an association between one candidate class and the image feature, the certainty level, or the probability data; wherein the method further comprises the steps of: modifying, by the controller, one or more parts of the cardiac image data; evaluating, by the trained machine learning data processing model, a change in the output data in response to the step of modifying, wherein the change relates to at least one of the candidate classes, the association, the certainty level, or the probability data; and identifying, based on the step of evaluation, one or more regions of significance in the cardiac image data; and providing region-indicating data, wherein the region-indicating data is indicative of the one or more regions of significance.

The above two groups of embodiments, which may be referred to as class activation mapping, provide significant advantages, because they enable to provide more insight in the image features and regions of an image that support a certain outcome. For example, certain image features may be more relevant to a certain outcome. By analysis of the weights in the above manner, it becomes apparent which image features are of greater weight in a certain outcome (e.g. why presence of certain image features indicates that a certain class does not appear to apply, or vice versa). These image features or regions in the image may then be highlighted in the output of the system, such as to present these to the user. In particular in those outcomes wherein the user is requested to provide further input to confirm or disaffirm the outcome, this may be helpful. However, also in the cases where the outcome is clear, the highlighting of these features may provide valuable information to the user. Therefore, in some of the above embodiments, the method further comprises providing output to a user of the system, wherein the output comprises the region-indicating data.

In yet further embodiments, the step of sampling comprises at least one of: up-sampling, such as bilinear up-sampling or interpolation; down-sampling, such as decimation. Up-sampling or down-sampling may be desired in order to match the resolution of the region-indicating data to that of the image. It may also be applied to locally improve the image resolution, for example in order to add auxiliary image features or markers, e.g. indicators, explanatory text, weighting values, etc.

In some embodiments, the step of obtaining the data indicative of the region of interest comprises at least one of: obtaining user input indicative of the region of interest; or performing a pre-processing filter operation for establishing the data indicative of the region of interest; or performing, using the or a further trained machine learning data processing model, a pre-processing image recognition step for establishing the data indicative of the region of interest. A region of interest may be obtained in various different ways. As may be appreciated, a user may provide a region of interest as input to the system. For example, the user may be interested in a specific region of an image wherein a lesion or defect may be suspected, or which is otherwise of interest for evaluation. However, in some embodiments, a pre-processing filter operation may automatically establish a region of interest. This could be useful in those cases wherein a region of interest is relatively well to establish using a computer implemented method or electronic means, for example: by applying an algorithm; by considering a specific part of a spectrum; by performing contour recognition based on contrast, or based on color or texture differences; or because a certain region of interest may always be found in a specific part of an image (this may be a fixed part of the image (e.g. always in the upper left corner, or in the middle) or in relation to other features (e.g. always at a certain location between two well recognizable lung areas). However, in some embodiments, the region of interest may be established performing, using the or a further trained machine learning data processing model, a pre-processing image recognition step for establishing the data indicative of the region of interest. Here, image recognition may be performed by the data processing model in order to perform the automatic step. Each of the latter two automatic processes provides the advantage of ease of use of the system, and further enables to perform the analysis for a series of images containing cardiac image data. For example, a collection of cardiac image data from a database may be examined in this manner in order to establish statistics on a certain physical condition, such as statistics on the occurrence of specific types of left atrial appendage (LAA) morphology: chicken-wing, windsock, cauliflower, and cactus.

In some embodiments, the cardiac image data comprises one or more of: computed tomography scan data, positron emission tomography data, magnetic resonance imaging data, ultrasound imaging data such as echocardiographic imaging data, radiographic imaging data, angiographic imaging data. In fact, the invention may be applied to any imaging modality, and the abovementioned modalities are mentioned mainly as examples.

In yet further embodiments, the region of interest is related to an atrium of the patient and the classes of the set of classes comprise aortic valve morphology labels. For example, in some embodiments, the aortic valve morphology labels comprise at least one or more of: a bicuspid aortic valve type 0 (BAV-0) label, a bicuspid aortic valve type 1 (BAV-1) label, a tricuspid aortic valve (TAV) label, quadricuspid aortic valve (QAV) label, pentacuspid aortic valve (PAV) label, unicuspid aortic valve (UAV) label, or n-cuspid aortic valve label.

In other or further embodiments, the method may be applied in order to evaluate various other characteristics and conditions. For example, in a method in accordance with such applications, one or more of the following may apply. In some embodiments, the region of interest is related to a left atrial appendage and the classes of the set of classes comprise left atrial appendage morphology labels, such as a cauliflower label, a chicken wing label, a windsock label or a cactus label. In a different application, the region of interest is related to a mitral valve of the patient and the classes of the set of classes comprise calcification grade labels, such as wherein the calcification grade labels comprise at least a mild label, a moderate label and a severe label. In these embodiments, other or further labels may be applied, such as percentage classes or certain ranges that indicate seriousness. In yet other embodiments, the region of interest is related to an aortic root of the patient and the classes of the set of classes comprise calcification grade labels, such as wherein the calcification grade labels comprise at least a mild label, a moderate label and a severe label. Also in these embodiments, other or further labels may be applied, such as percentage classes or certain ranges that indicate seriousness. In other or further embodiments, the cardiac characteristic relates to a cardiac phase and the classes of the set of classes comprise percentage intervals or percentage labels. In other or further embodiments, the region of interest is related to a left atrial appendage (LAA) and the cardiac characteristic relates to a left atrial appendage filling, wherein the classes of the set of classes comprise percentage intervals or percentage labels.

In accordance with a second aspect of the invention, there is provided herewith a method of training a machine learning data processing model for performing a method according to the first aspect, for classifying an image feature in order to determine, from cardiac image data, a cardiac characteristic of a patient by evaluating a probability that the image feature matches each class of a predetermined plurality of classes forming a set of classes such as to characterize the image feature, the method comprising: a) receiving, by the machine learning data processing model, a training data set comprising training data, the training data including cardiac image data of at least a part of a cardiac image, and optionally data indicative of a region of interest which includes the image feature; b) obtaining, by the machine learning data processing model, a plurality of classes forming a set of classes, wherein the classes together describe status classes of the cardiac characteristic; c) receiving, by the machine learning data processing model, a ground truth data set comprising ground truth data indicative of a ground truth class that matches with the image feature; d) training the machine learning data processing model based on the training data received in step a), the classes of the set of classes obtained in step b), and the ground truth data received in step c), for enabling the machine learning data processing model, after completion of the training period, to perform the step of providing probability data, the probability data comprising for each class of the set of classes a probability that the image feature matches the respective class.

The above method in accordance with the second aspect, enables to train a machine learning data processing model in order to use such a trained model in a method in accordance with the first aspect. The machine learning data processing model that may be trained using this method may be a neural network that includes a Gaussian layer, such as an artificial neural network or a convolutional neural network including a Gaussian layer, or a Bayesian network. These data processing models enable to provide probability date together with potential outcomes, such as applied in the method of the present invention.

In accordance with a third aspect of the invention, there is provided herewith an electronic image processing system for use in a method according to the first aspect, the system comprising a trained machine learning data processing model according to the second aspect, for classifying an image feature in order to determine, from cardiac image data, a cardiac characteristic of a patient by evaluating a probability that the image feature matches each class of a predetermined plurality of classes forming a set of classes such as to characterize the image feature, wherein the system further comprises one or more processors and a memory storing the trained machine learning data processing model and one or more instructions, which when loaded into the memory enable the one or more processors to perform the steps of: receiving, by the trained machine learning data processing model, the cardiac image data; obtaining, by the trained machine learning data processing model, data indicative of the region of interest; collecting, from the trained machine learning data processing model, probability data, the probability data comprising for each class of the set of classes a probability that the image feature matches the respective class; wherein the method further comprises, the steps of: evaluating, by a controller, the probability data for the classes of the set of classes, and identifying one or more candidate classes having an above average probability of matching with the image feature; determining a certainty level on the basis of the probability data for each of the identified candidate classes based on a comparison of the probabilities obtained in the evaluation step, wherein the certainty level is indicative of the certainty that the image feature matches a particular candidate class; and dependent on the step of determining, performing at least one step of: when the number of candidate classes equals one, and the certainty level exceeds a threshold level: associating the one candidate class with the image feature; or when the number of candidate classes equals one, and the certainty level is smaller than a threshold level: requesting user input indicative of a confirmation or disaffirmation that the one candidate class matches with the image feature; or when the number of candidate classes exceeds one: requesting, for each of the one or more candidate classes, user input indicative of a confirmation or disaffirmation that the respective candidate class matches with the image feature.

### Brief description of the drawings

The invention will further be elucidated by description of some specific embodiments thereof, making reference to the attached drawings. The detailed description provides examples of possible implementations of the invention, but is not to be regarded as describing the only embodiments falling under the scope. The scope of the invention is defined in the claims, and the description is to be regarded as illustrative without being restrictive on the invention. In the drawings:
Figure 1 schematically illustrates the artificial neural network for performing image recognition, in accordance with the prior art;
Figure 2 schematically illustrates a neural network including a Gaussian process layer for adding probability data to an image recognition process;
Figure 3 schematically illustrates the application of trained machine learning data processing model in an embodiment of the present invention;
Figure 4 schematically illustrates an embodiment of the method of the present invention;
Figure 5 schematically illustrates an embodiment of the method and system of the present invention;
Figure 6 schematically illustrates a method of the present invention in accordance with an embodiment thereof; and
Figure 7 schematically illustrates an electronic image processing system in accordance with an embodiment of the present invention.

### Detailed description of the drawings

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

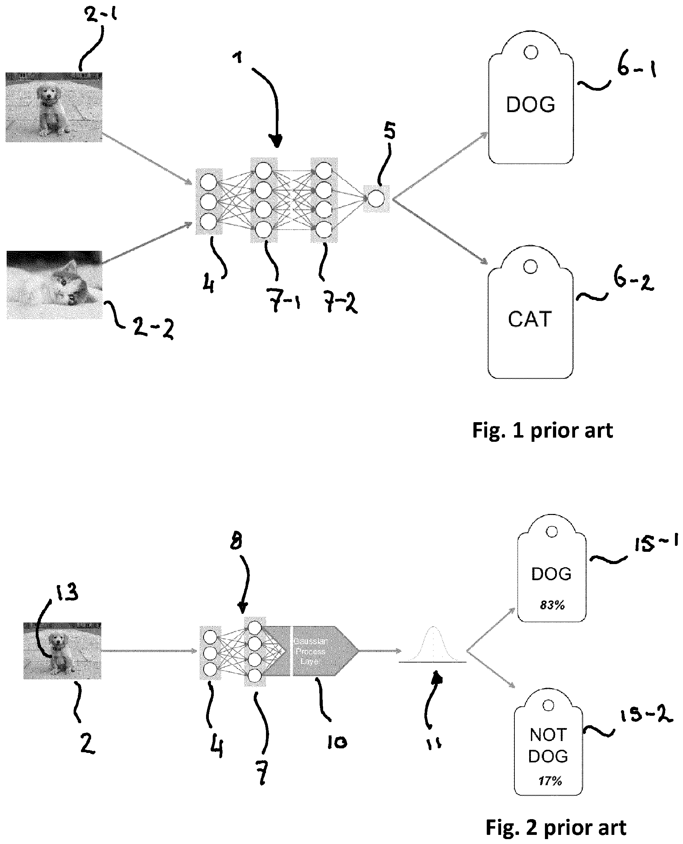

In Figure 1, a conventional artificial neural network 1 for performing image recognition is schematically illustrated. The neural network 1 comprises an input layer 4 and an output layer 5. At the input layer 4, the neural network 1 may receive various images 2-1 and 2-2. The neural network 1 in figure 1 has been trained to perform image recognition, and is able to recognize the subjects shown on each of the images 2-1 and 2-2 as animals and determine the type of animal. In particular, the neural network 1 may be trained to either identify the animal on the images 2-1 or 2-2 as a dog or a cat. To enable the neural network 1 to perform this task, the neural network 1 may have undercome a training program. The training program for example could have been a supervised training program. In such a training program. A plurality of various images 2 of dogs and cats may have been offered to the neural network 1 together with ground truth data. The ground truth data in this case provides the correct classification, i.e. the classification as provided or confirmed by an operator or user. During training, the images 2 may be provided to the neural network 1 via the input layer 4. In a first phase, the ground truth data may be actively used to adapt and optimize the parameters in the various layers (e.g. intermediate layers 7) of the neural network 1 such as to provide the correct animal type classification: 'dog' or 'cat'. In a second phase of the training, the neural network 1 receives the images 2 (such as 2-1 and 2-2) via the input layer 4 and provides a classification at the output layer 5. These classifications of the images 2 at the output 5 are then verified against the ground truth data. Based on a comparison of the results 6-1 and 6-2 coming from the output layer 5 and the ground truth data, the parameters in each of the intermediate layers 7-1 and 7-2 and their interconnections may be altered to train the network. Dependent on whether the classes provided as results 6-1 and 6-2 are correct, the neural network 1 may be optimized. After training, the conventional neural network 1 is then able to classify an animal on an arbitrary image 2 as being either a 'cat' or a 'dog'.

Although neural network 1 at the output layer 5 may provide impressive recognition results after proper training, the neural network 1 of figure 1 does not provide any insight into how certain this result is, or in other words, how likely it is that the neural network 1 provides an incorrect class as result 6 for an image 2. This in particular will become an issue in those areas wherein the differences between elements of two or more classes are only marginal, and where at the same time the risks involved in making an incorrect assessment may be significant. So, in the example of distinguishing between cats and dogs in arbitrary images the risks may be absent. However, the risks involved in distinguishing between classes in a medical setting may be significant, in particular when potentially life threatening physical conditions are involved.

Figure 2 illustrates another neural network 8 comprising an input layer 4, various intermediate layers 7 and a Gaussian process layer 10. Gaussian processes are a generalization of the Gaussian probability distribution. Gaussian process regression calculates the probability distribution over all admissible outcomes. The Gaussian process layer 10 thus enables the neural network 8 to provide after training, with each outcome, probability data. The probability data provided is indicative of a probability that the class provided with the animal in the image 2, a dog, is indeed correct. To do this, one of the intermediate layers 7 of the neural network 8 interconnects with the Gaussian process layer 10, and the intermediate results from the intermediate layer 7 are processed to provide the probability in accordance with a Gaussian profile 11. At the output of neural network 8, the image feature 13 is not only classified as a dog 15-1, but also probability data is provided which indicates the probability that the image feature 13 is indeed a dog. In the example, the available classes are either the class 'dog' 15-1, or the counter class 'not dog' 15-2.

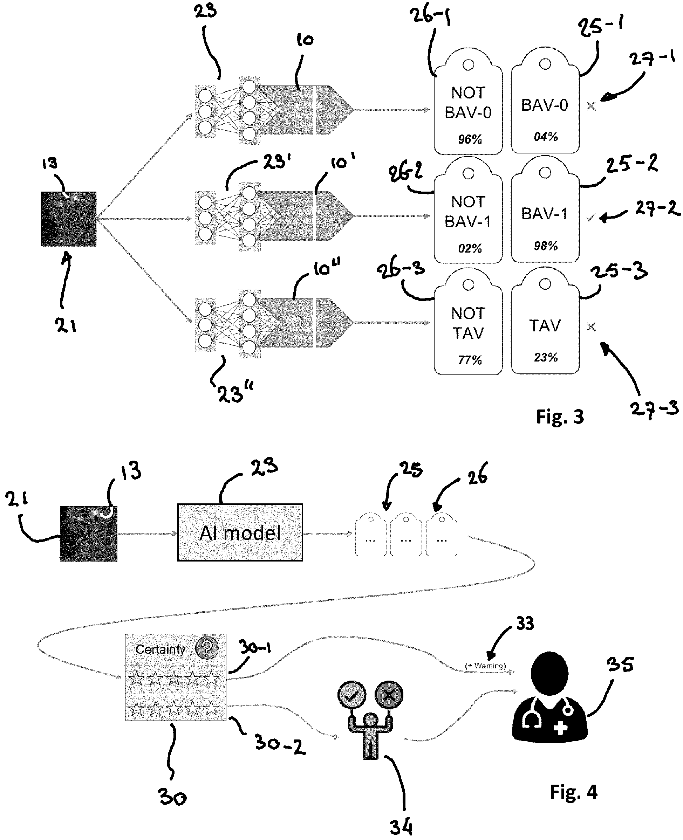

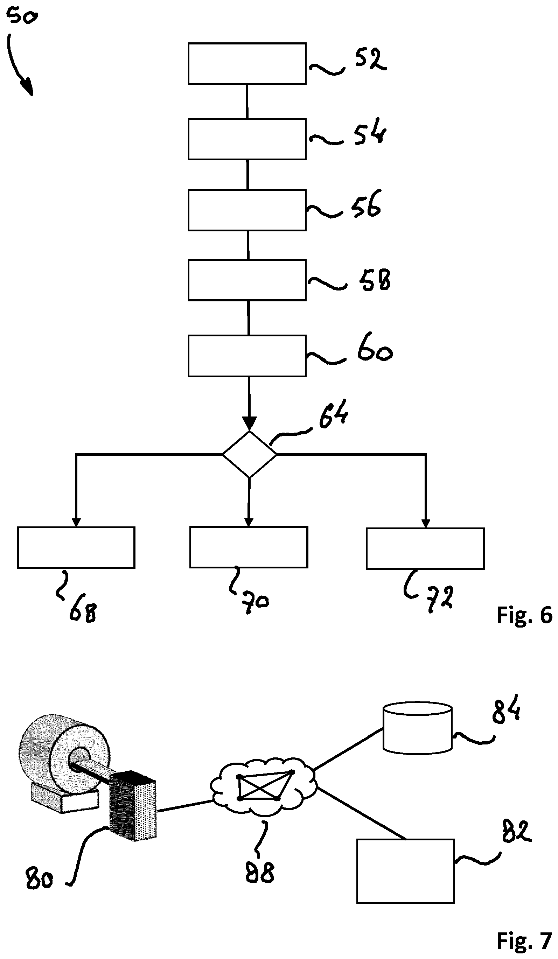

The present invention applies one or more Gaussian process layers 10 in a trained machine learning data processing model 23 (e.g. figure 3) that enables the classifying of image features from a cardiac image in order to determine therefrom a cardiac characteristic of a patient. For certain cardiac characteristics, the relevant image features 13 of images 21 obtained from an imaging system 80 (see figure 7) do not always provide 100% certainty as to how the imaged cardiac characteristic is to be classified. In these cases, making an incorrect classification may provide a direct serious health risk to the patient. The method and system of the present invention support a proper conclusion or diagnose by taking part of the uncertainty away, or otherwise making the user 35 (see figure 4) aware of this uncertainty and the level of uncertainty. In figure 3, the method of the present invention, which involves the use of a trained machine learning data processing model 23 is schematically illustrated. In the method illustrated in figure 3, the machine learning data processing model 23 is illustrated three times as 23, 23'and 23". In principal it is possible to use three different neural networks with Gaussian process layers 10, 10' and 10" that are each trained to evaluate a specific class (e.g. the 'bicuspid aortic valve type 0 (BAV-0)') and its counter-class (in that case 'NOT BAV-0'), in order to for the combination of data processing models 23, 23' and 23" to allow an evaluation as to whether or not the image feature illustrated in image 21 belongs to a specific class 25-1 (or 25-2 or 25-3), or its counter class 26-1 (or 26-2 or 26-3). However, this may also be achieved by training a single neural network 23 with one or more Gaussian layers 10 to perform the classification over all classes 25-1, 25-2 and 25-3 and/or their counter classes 26-1, 26-2 and 26-3. The classes in figure 3 are mutually exclusive, meaning that if the condition illustrated in image 21 is indeed of bicuspid aortic valve type 0 (BAV-0), it cannot be bicuspid aortic valve type 1 (BAV-1) nor tricuspid aortic valve (TAV). This is not a necessity for the method of the present invention, as one may appreciate it is also possible to add another class which is indicative of e.g. a severeness of a certain condition or a comorbidity. However, in order to not unnecessarily complicate the example, we will assume here that the classes 25-1, 25-2 and 25-3 are mutually exclusive. For each class 25-1 through 25-3, there is a counter class 26-1 through 26-3 indicative of the image feature not belonging to the respective class.

The system of figure 3 and its output provides three classes 25-1 through 25-3 and three counter classes 26-1 through 26-3, including their probability data of whether or not the class or counter class applies to the respective image feature. For example in image 21 of figure 3, there is a 96% probability that the image feature does not belong to the class BAV-0 indicative of an aortic valve morphology of bicuspid aortic valve type 0. The system provides a 98% probability that the image feature in image 21 belongs to the class bicuspid aortic valve type 1 (BAV-1). Furthermore, the system indicates that there is a 23% probability that the image feature in image 21 belongs to the class tricuspid aortic valve (TAV). Based on the probability data in each of the classes 25-1 through 25-3 and each of the counter classes 26-1 through 26-3, it is relatively certain that the image feature is visible in image 21 belongs to this particular class BAV-1. This will therefore be the result 27-2 presented at the output of figure 3. Other possibilities 27-1 and 27-3 are discarded. To perform this step, the system in figure 3 does determine a certainty level based on a comparison of the probabilities obtained in the evaluation step, wherein the certainty level is indicative of the certainty that the image feature in image 21 matches a particular class 25 or counter class 26, and on basis of that, because there is only as single candidate class (BAV-1) having a certainty level of 98%, whereas the nearest alternative has a probability of 23% (TAV). The difference between these two being 75% exceeds a preset threshold level (which may be set to any desired value between 1% and 100%, e.g. 20%, 30%, 40%, 50%, 60%, 65%, 70%, 75%, 80%, 85%, 90% or 95%), and therefore the system is able to directly associate the image feature in image 21 with the candidate class BAV-1. Alternatively, the system may also be programed to select the class having the highest probability if that probability at least is above a predetermined threshold level (for example a value in a range between 60% and 100%, or 80% and 100%). This of course may not always be the case.

Suppose the probability data in figure 3 would have been different, and suppose that the probability for class BAV-1 would have been 60% whereas the probability for class BAV-0 would have been 40%, it is still more likely that class BAV-1 will apply to the image feature of image 21, but the certainty level is much lower. The difference between probability is only 20% and the highest probability is at 60%. Therefore, the certainty level which is indicative of how certain it is that the suggested class at the output 5 is the correct class, may be below a predetermined threshold level. The threshold level may for example be any of the above, e.g. associated with a difference in the two (or more) highest probabilities or the minimal probability value of the class having the highest probability. Suppose such a probability difference threshold level would be set at a minimum of 30%, the certainty level in this example is not sufficient. The certainty level and the threshold in the invention serve as a safety measure in the system. If, as in this case, the certainty level is insufficient, a user 35 may be consulted to confirm or disaffirm the result, i.e. whether or not the class BAV-1 is indeed correct. This is illustrated in figure 4.

In figure 4, image 21 is offered at the input of the trained machine learning data processing model 23 (our convolutional neural network with Gaussian process layer 10). The data processing model 23 provides the classes 25 and counter classes 26 including probability data, as is illustrated in figure 3. Thereafter, the certainty level 30 of the result coming from data processing model 23 is determined. In the example of figure 4, the certainty level is represented as a number of stars between zero and five stars. The threshold level in such embodiments may likewise be set as a number of stars (e.g. three stars). If there is a sufficiently high certainty level as in situation 30-1, the method continues by providing the result of the classification directly to the user 35. This presentation of the result may include a warning 33 to the user 35, that the recognition is performed using an artificial intelligence model, and not by a physician. If the certainty level 30-2 is insufficient (here below three stars), user input 34 is requested in order to confirm or disaffirm that the candidate class suggested by the system 23 matches with the image feature 21. In that manner, the user 35 which may be a physician is informed of the fact that the system 23 considers the image feature to belong to a certain class, but the physician is also informed of the fact that the system 23 is not sufficiently certain about this. This therefore flags to physician 35 that additional care should be taken in evaluating this image or taking over the class 25 suggested by the system.

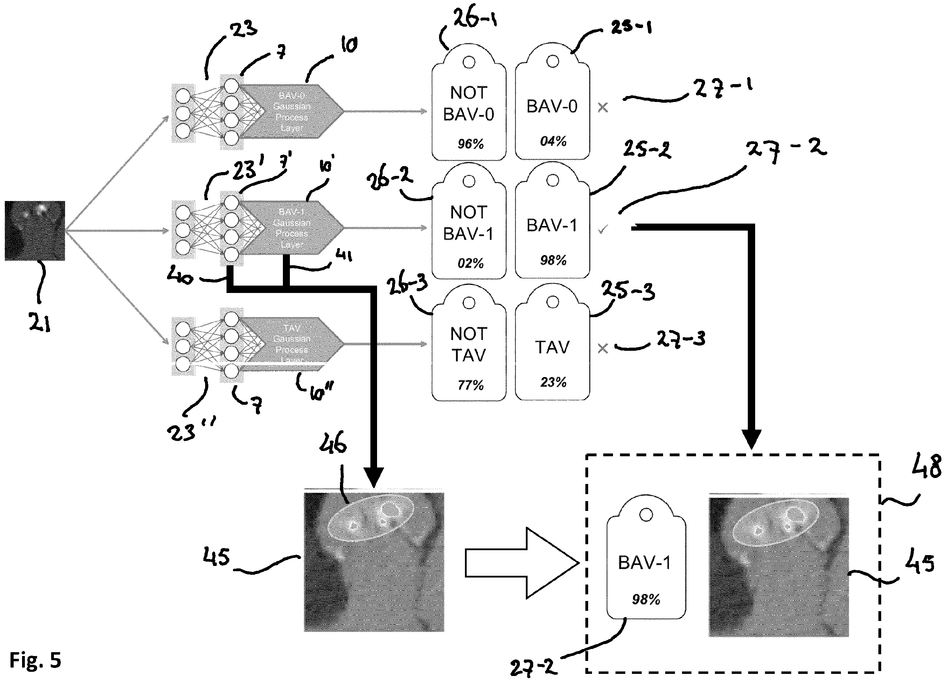

Figure 5 illustrates another embodiment of the method and system of the present invention, which provides the user 35 with even more information on the selected class. In figure 5, the system of figure 3 is schematically illustrated including again the machine learning data processing model 23, 23' and 23". In figure 5 a class activation mapping process is performed by analyzing the parameter values from intermediate layers 7' in step 40, as well as analyzing the Gaussian layer 10' in step 41. In particular, the class activation mapping is performed by obtaining from the intermediate layer 7' a weighting factor of a respective channel or interconnection present in the intermediate layer 7' and the intermediate data of N-channels in the intermediate layer 7'. The intermediate data from the channels is weighted using the weights of the intermediate layer 7', and optionally the data from the Gaussian process layer 10' may be used in order to identify the weighted intermediate data having a significant effect (e.g. the most effect) on the outcome of the classification process. This data may then be mapped onto the image 21, which yields the mapping 45 to include region indicating data, wherein those parts of the image 21 that are most significant in determining the resulting class have been highlighted. The mapping 45 is provided together with a result 27-2 and probability data as the outcome 48 of the system.

Figure 5 shows one category of embodiments that enables implementation of class activation mapping in the method of the invention. However, the invention is not limited to this particular implementation. Alternatively, class activation mapping may also be performed by examining the outcome of the model on characteristics of the input. For example by modifying input characteristics (e.g. relating to parts of a cardiac image) and evaluating how strongly the output responds to a change at the input. Such a method may for example be perturbation or occlusion-based. These methods evaluate how a predicted output class, segmentation or any of the intermediate outputs (feature maps/activation maps) change in response to occlusion or perturbation of a certain region. The magnitude of change relates to the importance of a given region. The region can be color-coded accordingly to yield visualizations. In this case no rescaling/sampling has to take place, the regions are defined in the original input image/volume. Examples are SHAP or LIME. Alternatively, same may be achieved based on or using saliency maps. These methods take a look at the gradient of the classification loss with respect to the input pixels/voxels. Highly salient pixels/voxels need to change the least to affect the class prediction the most. No rescaling/sampling has to take place here, a value is obtained for each pixel/voxel individually. There are extensions that multiply the gradients by the input value ('gradient * input') or that combine saliency maps with perturbation/occlusion-based methods. For instance, 'integrated gradients' compute the gradients for each input pixel/voxel in a set of increasingly less perturbed versions of the same image, as show in in the example below. The average of the input pixel/voxel gradients is computed to get the final result.

Back to figure 5 (although the below also applies to the alternatives, *mutatis mutandis*), the class activation mapping provides the user 35 with additional information as to how the system 23 came to the conclusion that the respective image 21 shows a BAV-1 type of aortic valve morphology. This enables the user 35 to evaluate this choice, and particular where the certainty level is low, allows the user 35 to reconsider this choice of class.

The above method illustrated in figure 5 is based on analysis of the values of the convolutional neural network. A convolutional neural network consists of a series of layers. An input image passes through the first layer, which results in feature maps. Next, some kind of pooling operation can take place which can reduce the dimensionality of the feature maps. The feature map gets passed on to other layers resulting in new feature maps at increasingly coarser resolutions. In a traditional CAM implementation, the penultimate feature maps are used, along with the weights of the final layer. The weights that are connected to the predicted class are multiplied with the different channels of the penultimate feature maps. These are the weights that resulted in the highest probability for this particular image to belong to class c. The result of the weighting is a class activation map which typically is of coarser resolution than the original image. To get a result in the original resolution, some resizing/upsampling has to take place. Note that in this implementation, an approach is used with GAP (global average pooling) which reduces each of the N feature maps to a single average value. These single values are multiplied with weights, resulting in N weights. Grad-CAM: Grad-CAM differs from CAM in the sense that is does not use raw layer weights, but rather uses the gradients. Grad-CAM is more flexible and computes the gradients of the classification loss for predicted class c with respect to each element of each feature map. An averaging step takes place over each feature map to give a single weighting value for each feature map. The average of the averaged feature is computed to give a visual output. Extensions exist to this approach that compute gradients on perturbed feature maps, or that combine CAM-like methods with saliency-based methods.

It is stressed that, although the above examples have been directed at the application of determining a type of aortic valve morphology, this is certainly not the only field of application wherein the above may be applied. The invention may likewise be applied in relation to different physical cardiac characteristics. For example, in the classification of left atrial appendage morphology, or calcification grade of an aortic root or a mitral valve. For example, the left atrial appendage morphology classes to be evaluated may include any one or more or all of: a cauliflower label, a chicken wing label, a windsock label or a cactus label. Instead or in addition, the classes may relate to parameter ranges, percentages, quantitative or qualitative descriptors, or the like. For example to express a calcification grade, it is possible to classify this as mild, moderate or severe, or otherwise e.g. in terms of vessel blocking percentage (e.g. percentage classes) or size range classes.

Figure 6 schematically illustrates a method in accordance with the present invention. In figure 6, a trained machine learning data processing model 23 receives cardiac image data 21 in step 52. For example this may be a cardiac image 21 illustrated in figure 3. In step 54 of method 50, the trained machine learning data processing model 23 may further receive data which is indicative of a region of interest. For example, user 35 may indicate that she/he is interested in a region that illustrates the aortic valve in order to make an assessment on the aortic valve morphology. The user 35 may simply indicate the respective region by indicating or marking the contours thereof. Alternatively, if possible, an automatic algorithm or preprocessing filter operation may be performed in order to identify the region of interest and provide this to the system 23. As a further alternative, it is also possible to apply a further trained machine learning data processing model which is able to recognize a specific region of interest in a cardiac image and automatically provide the region of interest to the trained machine learning data processing model 23. For example a further trained machine learning data processing model may be able to recognize the location of the aortic valves, or any of the cardiac atriums, or the aortic root valve or the like.

In step 56, from the trained machine learning data processing model 23, probability data is collected from the Gaussian process layer 10. This probability data provides, for each class 25 of the set of classes to be considered, a probability that the image feature matches respective class. Optionally, it is further possible to add counter classes 26 to each class 25, indicative of the probability that an image feature does not match a specific class.

In step 58, the outcome from trained machine learning data processing model 23 is evaluated by the controller. The controller evaluates probability data for each class 25 of the set of classes, and from this identifies one or more classes having an above averaged probability of matching with the specific image feature. Then, in step 60 a certainty level is determined on the basis of the probability data for each of the identified classes. The certainty level is indicative of the certainty that the image feature matches a certain preferred class 25 of the set of classes. For example, the certainty level may be high or low dependent on whether or not the probability data provides a large probability for certain class and thereby a large difference between probabilities of the preferred class with respect to the other classes. In step 64, the outcome of the determining step is evaluated. In case where there is only one candidate class left, and the certainty level is high, such as in figure 3, the method continuous in step 68 by directly associating this one candidate class with the image feature from the image 21. Alternatively, in step 70, if there is one candidate class and the certainty level is below a threshold level (for example a probability of 60% in favor of the class, while the probabilities of the other classes are around 30%), the system requests user input in order to indicate whether the preferred class is confirmed or disaffirmed by the user 35. In any case where there is more than one candidate class left, in step 72 the user 35 is requested, for each of these candidate classes, to indicate a confirmation of disaffirmation that the respective class 25 matches with the image feature.

Figure 7 schematically illustrates a system in accordance with the present invention. In figure 7, an imaging system 80 is schematically illustrated which is connected to an electronic image processing system 82 via a data network 88. The data network 88 may be a local area network of a hospital, for example. Furthermore, the images from the imaging system 80 may have been stored in the database 84, from which the electronic image processing system 82 may also retrieve images 21 in order to perform the processing thereof using the method of the present invention.

The present invention has been described in terms of some specific embodiments thereof. It will be appreciated that the embodiments shown in the drawings and described herein are intended for illustrated purposes only and are not by any manner or means intended to be restrictive on the invention. It is believed that the operation and construction of the present invention will be apparent from the foregoing description and drawings appended thereto. It will be clear to the skilled person that the invention is not limited to any specific embodiment herein described and that modifications are possible which should be considered within the scope of the appended claims.

In the claims, any reference signs shall not be construed as limiting the claim. The term 'comprising' and 'including' when used in this description or the appended claims should not be construed in an exclusive or exhaustive sense but rather in an inclusive sense. Thus the expression 'comprising' as used herein does not exclude the presence of other elements or steps in addition to those listed in any claim. Expressions such as "consisting of", when used in this description or the appended claims, should be construed not as an exhaustive enumeration but rather in an inclusive sense of "at least consisting of". Furthermore, the words 'a' and 'an' shall not be construed as limited to 'only one', but instead are used to mean 'at least one', and do not exclude a plurality. Features that are not specifically or explicitly described or claimed may be additionally included in the structure of the invention within its scope. Any of the claimed or disclosed devices or portions thereof may be combined together or separated into further portions unless specifically stated otherwise, without departing from the claimed invention. Expressions such as: "means for ..." should be read as: "component configured for ..." or "member constructed to ..." and should be construed to include equivalents for the structures disclosed. The use of expressions like: "critical", "preferred", "especially preferred" etc. is not intended to limit the invention. The invention may be practiced otherwise then as specifically described herein, and is only limited by the appended claims.

## Claims

1. In an electronic image processing system (82), a method of classifying an image feature (13) in order to determine, from cardiac image data (21), a cardiac characteristic of a patient by using a trained machine learning data processing model (23, 23', 23"), wherein the trained machine learning data processing model is trained to evaluate a probability that the image feature matches each class of a predetermined plurality of classes forming a set of classes such as to characterize the image feature, the cardiac image data comprising at least a region of interest including the image feature, wherein the method comprises:
receiving (52), by the trained machine learning data processing model, the cardiac image data;
obtaining (54), by the trained machine learning data processing model, data indicative of the region of interest;
collecting (56), from the trained machine learning data processing model, probability data, the probability data comprising for each class (25, 26) of the set of classes a probability that the image feature matches the respective class;
wherein the method further comprises, the steps of:
evaluating (58), by a controller, the probability data for the classes of the set of classes, and identifying one or more candidate classes having a significant probability of matching with the image feature;
determining (60) a certainty level (30) on the basis of the probability data for each of the identified candidate classes based on a comparison of the probabilities obtained in the evaluation step, wherein the certainty level is indicative of the certainty that the image feature matches a particular candidate class; and
dependent on the step of determining, performing at least one step of:
when the number of candidate classes equals one, and the certainty level exceeds a threshold level: associating (68) the one candidate class with the image feature; or
when the number of candidate classes equals one, and the certainty level is smaller than a threshold level: requesting (70) user input (34) indicative of a confirmation or disaffirmation that the one candidate class matches with the image feature; or
when the number of candidate classes exceeds one: requesting (72), for each of the one or more candidate classes, user input indicative of a confirmation or disaffirmation that the respective candidate class matches with the image feature.

2. Method according to claim 1, further comprising providing output to a user (35) of the system, wherein the output comprises at least one element of a group comprising: data indicative of the one or more candidate classes; data of an association between one candidate class and the image feature; the certainty level; or the probability data.

3. Method according to claim 1 or 2, wherein for providing the probability data, the trained machine learning data processing model comprises a Gaussian layer (10, 10') in order to determine for each class of the set of classes said probability that the image feature matches the respective class.

4. Method according to any one or more of the preceding claims, wherein the trained machine learning data processing model is a convolutional neural network (1, 8) including one or more intermediate layers (7, 7'), and wherein the step of collecting further comprises:
obtaining from at least one of the intermediate layers, a weighting vector of M weights and intermediate data of M channels, wherein M comprises a natural number larger than zero;
weighting the intermediate data using the weights resulting in weighted intermediate data; and
sampling the weighted intermediate data resulting in region-indicating data, the region-indicating data being indicative of one or more regions of significance of the cardiac image having an above average weighing in the weighted intermediate data.

5. Method according to any one or more of the claims 1-3, wherein the trained machine learning data processing model comprises an input layer (4) for receiving at least the cardiac image data, and an output layer (5) for providing output data, the output data including at least one of: data indicative of the one or more candidate classes, an association between one candidate class and the image feature, the certainty level, or the probability data;
wherein the method further comprises the steps of:
modifying, by the controller, one or more parts of the cardiac image data;
evaluating, by the trained machine learning data processing model, a change in the output data in response to the step of modifying, wherein the change relates to at least one of the candidate classes, the association, the certainty level, or the probability data; and
identifying, based on the step of evaluation, one or more regions of significance in the cardiac image data; and
providing region-indicating data, wherein the region-indicating data is indicative of the one or more regions of significance.

6. Method according to claim 4 or 5, further comprising providing output to a user of the system, wherein the output comprises the region-indicating data.

7. Method according to any one or more of claims 4-6, wherein the step of sampling comprises at least one of: up-sampling, such as bilinear up-sampling or interpolation; down-sampling, such as decimation.

8. Method according to one or more of the preceding claims, wherein the step of obtaining the data indicative of the region of interest comprises at least one of:
obtaining user input indicative of the region of interest; or
performing a pre-processing filter operation for establishing the data indicative of the region of interest; or
performing, using the or a further trained machine learning data processing model, a pre-processing image recognition step for establishing the data indicative of the region of interest.

9. Method according to any one or more of the preceding claims, wherein the cardiac image data comprises one or more of: computed tomography scan data, positron emission tomography data, magnetic resonance imaging data, ultrasound imaging data such as echocardiographic imaging data, radiographic imaging data, angiographic imaging data.

10. Method according to any one or more of the preceding claims, wherein the region of interest is related to an atrium of the patient and the classes of the set of classes comprise aortic valve morphology labels.

11. Method according to claim 10, wherein the aortic valve morphology labels comprise at least one or more of: a bicuspid aortic valve type 0 label, a bicuspid aortic valve type 1 label, a tricuspid aortic valve label, quadricuspid aortic valve label, pentacuspid aortic valve label, unicuspid aortic valve label, or n-cuspid aortic valve label.

12. Method according to any one or more of the claims 1-9, wherein at least one of:
the region of interest is related to a left atrial appendage and the classes of the set of classes comprise left atrial appendage morphology labels, such as a cauliflower label or a chicken wing label; or
the region of interest is related to a mitral valve of the patient and the classes of the set of classes comprise calcification grade labels, such as wherein the calcification grade labels comprise at least a mild label, a moderate label and a severe label; or
the region of interest is related to an aortic root of the patient and the classes of the set of classes comprise calcification grade labels, such as wherein the calcification grade labels comprise at least a mild label, a moderate label and a severe label; or
the cardiac characteristic relates to a cardiac phase and the classes of the set of classes comprise percentage intervals or percentage labels;
the region of interest is related to a left atrial appendage and the cardiac characteristic relates to a left atrial appendage filling, wherein the classes of the set of classes comprise percentage intervals or percentage labels.

13. Method of training a machine learning data processing model (23, 23', 23"), wherein the machine learning data processing model is configured for performing a method according to any one or more of the preceding claims, for classifying an image feature (13) in order to determine, from cardiac image data (21), a cardiac characteristic of a patient by evaluating a probability that the image feature matches each class (25, 26) of a predetermined plurality of classes forming a set of classes such as to characterize the image feature, the method of training comprising:
a) receiving, by the machine learning data processing model, a training data set comprising training data, the training data including cardiac image data of at least a part of a cardiac image, and optionally data indicative of a region of interest which includes the image feature;
b) obtaining, by the machine learning data processing model, a plurality of classes forming a set of classes, wherein the classes together describe status classes of the cardiac characteristic;
c) receiving, by the machine learning data processing model, a ground truth data set comprising ground truth data indicative of a ground truth class that matches with the image feature;
d) training the machine learning data processing model based on the training data received in step a), the classes of the set of classes obtained in step b), and the ground truth data received in step c), for enabling the machine learning data processing model, after completion of the training period, to perform the step of providing probability data, the probability data comprising for each class of the set of classes a probability that the image feature matches the respective class.

14. An electronic image processing system (82) for use in a method according to any one or more of the claims 1-12, the system comprising a trained machine learning data processing model (23, 23', 23") according to claim 13, for classifying an image feature (13) in order to determine, from cardiac image data, a cardiac characteristic of a patient by evaluating a probability that the image feature matches each class (25, 26) of a predetermined plurality of classes forming a set of classes such as to characterize the image feature, the cardiac image data comprising at least a region of interest including the image feature, wherein the system further comprises one or more processors and a memory storing the trained machine learning data processing model and one or more instructions, which when loaded into the memory enable the one or more processors to perform the steps of:
receiving, by the trained machine learning data processing model, the cardiac image data;
obtaining, by the trained machine learning data processing model, data indicative of the region of interest;
collecting, from the trained machine learning data processing model, probability data, the probability data comprising for each class of the set of classes a probability that the image feature matches the respective class;
wherein the method further comprises, the steps of:
evaluating, by a controller, the probability data for the classes of the set of classes, and identifying one or more candidate classes having a significant probability of matching with the image feature;
determining a certainty level on the basis of the probability data for each of the identified candidate classes based on a comparison of the probabilities obtained in the evaluation step, wherein the certainty level is indicative of the certainty that the image feature matches a particular candidate class; and
dependent on the step of determining, performing at least one step of:
when the number of candidate classes equals one, and the certainty level exceeds a threshold level: associating the one candidate class with the image feature; or
when the number of candidate classes equals one, and the certainty level is smaller than a threshold level: requesting user input indicative of a confirmation or disaffirmation that the one candidate class matches with the image feature; or
when the number of candidate classes exceeds one: requesting, for each of the one or more candidate classes, user input indicative of a confirmation or disaffirmation that the respective candidate class matches with the image feature.

## Patentansprüche

1. Ein in einem elektronischen Bildverarbeitungssystem (82) befindliches Verfahren zum Klassifizieren eines Bildmerkmals (13), um aus Herzbilddaten (21) eine Herzcharakteristik eines Patienten zu bestimmen, unter Verwendung eines trainierten Datenverarbeitungsmodells (23, 23', 23") für maschinelles Lernen, wobei das trainierte Datenverarbeitungsmodell für maschinelles Lernen trainiert wird, um eine Wahrscheinlichkeit zu bewerten, dass das Bildmerkmal mit jeder Klasse einer vorbestimmten Vielzahl von Klassen übereinstimmt, die einen Klassensatz bilden, um so das Bildmerkmal zu charakterisieren, wobei die Herzbilddaten mindestens einen Untersuchungsbereich umfassen, der das Bildmerkmal enthält, wobei das Verfahren Folgendes umfasst:
Empfangen (52) der Herzbilddaten durch das trainierte Datenverarbeitungsmodell für maschinelles Lernen;
Erhalten (54) von Daten, die für den Untersuchungsbereich bezeichnend sind, durch das trainierte Datenverarbeitungsmodell für maschinelles Lernen;
Sammeln (56) von Wahrscheinlichkeitsdaten aus dem trainierten Datenverarbeitungsmodell für maschinelles Lernen, wobei die Wahrscheinlichkeitsdaten für jede Klasse (25, 26) des Klassensatzes eine Wahrscheinlichkeit umfassen,
dass das Bildmerkmal mit der jeweiligen Klasse übereinstimmt;
wobei das Verfahren ferner die folgenden Schritte umfasst:
Auswerten (58) der Wahrscheinlichkeitsdaten für die Klassen des Klassensatzes durch eine Steuerung und Identifizieren einer oder mehrerer Kandidatenklassen mit einer signifikanten Wahrscheinlichkeit der Übereinstimmung mit dem Bildmerkmal;
Bestimmen (60) eines Gewissheitsgrades (30) auf Grundlage der Wahrscheinlichkeitsdaten für jede der identifizierten Kandidatenklassen auf der Grundlage eines Vergleichs der in dem Auswertungsschritt erhaltenen Wahrscheinlichkeiten, wobei der Gewissheitsgrad die Gewissheit angibt, dass das Bildmerkmal mit einer bestimmten Kandidatenklasse übereinstimmt; und
in Abhängigkeit von dem Schritt des Bestimmens mindestens eines der folgenden Schritte:
wenn die Anzahl der Kandidatenklassen gleich eins ist und der Gewissheitsgrad einen Schwellenwert überschreitet:
Assoziieren (68) der einen Kandidatenklasse mit dem Bildmerkmal; oder
wenn die Anzahl der Kandidatenklassen gleich eins ist und der Gewissheitsgrad kleiner als ein Schwellenwert ist:
Anfordern (70) einer Benutzereingabe (34), die eine Bestätigung oder Nichtbestätigung der Übereinstimmung der einen Kandidatenklasse mit dem Bildmerkmal anzeigt; oder
wenn die Anzahl der Kandidatenklassen größer als eins ist:
Anfordern (72) einer Benutzereingabe für jede der einen oder mehreren Kandidatenklassen, die eine Bestätigung oder Nichtbestätigung der Übereinstimmung der jeweiligen Kandidatenklasse mit dem Bildmerkmal anzeigt.

2. Verfahren nach Anspruch 1, das ferner das Bereitstellen einer Ausgabe an einen Benutzer (35) des Systems umfasst, wobei die Ausgabe mindestens ein Element einer Gruppe umfasst, die Folgendes umfasst:
Daten, die für die eine oder mehrere Kandidatenklassen bezeichnend sind; Daten über eine Assoziation zwischen einer Kandidatenklasse und dem Bildmerkmal; den Gewissheitsgrad; oder die Wahrscheinlichkeitsdaten.

3. Verfahren nach Anspruch 1 oder 2, wobei zur Bereitstellung der Wahrscheinlichkeitsdaten das trainierte Datenverarbeitungsmodell für maschinelles Lernen eine Gaußsche Schicht (10, 10') umfasst, um für jede Klasse des Klassensatzes die Wahrscheinlichkeit zu bestimmen, dass das Bildmerkmal mit der jeweiligen Klasse übereinstimmt.

4. Verfahren nach einem oder mehreren der vorhergehenden Ansprüche, wobei das trainierte Datenverarbeitungsmodell für maschinelles Lernen ein faltendes neuronales Netzwerk (1, 8) ist, das eine oder mehrere Zwischenschichten (7, 7') enthält, und wobei der Schritt des Sammelns ferner Folgendes umfasst:
Erhalten eines Gewichtungsvektors von M Gewichten und Zwischendaten von M Kanälen von mindestens einer der Zwischenschichten, wobei M eine natürliche Zahl größer als Null ist;
Gewichtung der Zwischendaten unter Verwendung der Gewichte, was zu gewichteten Zwischendaten führt; und
Abtasten der gewichteten Zwischendaten, was zu bereichsbezeichnenden Daten führt, wobei die bereichsbezeichnenden Daten auf einen oder mehrere signifikante Bereiche des Herzbildes hinweisen, die ein überdurchschnittliches Gewicht in den gewichteten Zwischendaten aufweisen.

5. Verfahren nach einem oder mehreren der Ansprüche 1 bis 3, wobei das trainierte Datenverarbeitungsmodell für maschinelles Lernen eine Eingabeschicht (4) zum Empfangen mindestens der Herzbilddaten und eine Ausgabeschicht (5) zum Bereitstellen von Ausgabedaten umfasst, wobei die Ausgabedaten mindestens eines der folgenden Elemente enthalten:
Daten, die auf die eine oder mehrere Kandidatenklassen hinweisen, eine Assoziation zwischen einer Kandidatenklasse und dem Bildmerkmal, der Gewissheitsgrad oder die Wahrscheinlichkeitsdaten;
wobei das Verfahren ferner die folgenden Schritte umfasst:
Modifizieren eines oder mehrerer Teile der Herzbilddaten durch die Steuerung;
Auswerten einer Änderung in den Ausgabedaten als Reaktion auf den Schritt des Modifizierens durch das trainierte Datenverarbeitungsmodell für maschinelles Lernen, wobei sich die Änderung auf mindestens eine der Kandidatenklassen, die Assoziation, den Gewissheitsgrad oder die Wahrscheinlichkeitsdaten bezieht; und
Identifizieren, basierend auf dem Schritt der Auswertung, einer oder mehrerer signifikanter Bereiche in den Herzbilddaten; und
Bereitstellen von bereichsbezeichnenden Daten, wobei die bereichsbezeichnenden Daten auf die eine oder mehrere signifikante Bereiche hinweisen.

6. Verfahren nach Anspruch 4 oder 5, das ferner das Bereitstellen einer Ausgabe an einen Benutzer des Systems umfasst, wobei die Ausgabe die bereichsbezeichnenden Daten umfasst.

7. Verfahren nach einem oder mehreren der Ansprüche 4 bis 6, wobei der Schritt des Abtastens mindestens einen der folgenden Schritte umfasst:
Abtastratenerhöhung, wie z. B. bilineare Abtastratenerhöhung oder Interpolation; Downsampling, z. B. Dezimierung.

8. Verfahren nach einem oder mehreren der vorhergehenden Ansprüche, wobei der Schritt des Erhaltens der Daten, die auf den Untersuchungsbereich hinweisen, mindestens einen der folgenden Schritte umfasst:
Erhalten einer Benutzereingabe, die für den Untersuchungsbereich bezeichnend ist; oder
Durchführen einer Vorverarbeitungs-Filteroperation zur Ermittlung der Daten, die auf den Untersuchungsbereich hinweisen; oder
Durchführen eines Vorverarbeitungsschritts zur Bilderkennung unter Verwendung des oder eines weiteren trainierten Datenverarbeitungsmodells für maschinelles Lernen, um die Daten zu ermitteln, die auf den Untersuchungsbereich hinweisen.

9. Verfahren nach einem oder mehreren der vorhergehenden Ansprüche, wobei die Herzbilddaten eines oder mehrere der folgenden Elemente umfassen:
Computertomographiescandaten, Positronenemissionstomographiedaten, Magnetresonanztomographiedaten, Ultraschallbilddaten wie Echokardiographiedaten, Röntgenbilddaten, Angiographiedaten.

10. Verfahren nach einem oder mehreren der vorhergehenden Ansprüche, wobei sich der Untersuchungsbereich auf einen Vorhof des Patienten bezieht und die Klassen des Klassensatzes Aortenklappenmorphologie-Labels umfassen.

11. Verfahren nach Anspruch 10, wobei die Aortenklappenmorphologie-Labels mindestens eines oder mehrere der folgenden Labels umfassen:
ein Label der bikuspiden Aortenklappe Typ 0, ein Label der bikuspiden Aortenklappe Typ 1, ein Label der trikuspiden Aortenklappe, ein Label der quadrikuspiden Aortenklappe, ein Label der pentakuspiden Aortenklappe, ein Label der unikuspiden Aortenklappe oder ein Label der n-kuspiden Aortenklappe.

12. Verfahren nach einem oder mehreren der Ansprüche 1 bis 9, wobei mindestens eines der folgenden erfüllt ist:
der Untersuchungsbereich steht mit einem linken Vorhofanhang in Beziehung und die Klassen des Klassensatzes umfassen Morphologie-Labels für den linke Vorhofanhang, wie beispielsweise ein Blumenkohl-Label oder ein Hühnerflügel-Label;
der Untersuchungsbereich bezieht sich auf eine Mitralklappe des Patienten und die Klassen des Klassensatzes umfassen Verkalkungsgrad-Labels, wobei die Verkalkungsgrad-Labels mindestens ein leichtes Label,
ein mittleres Label und ein schweres Label umfassen;
der Untersuchungsbereich bezieht sich auf eine Aortenwurzel des Patienten und die Klassen des Klassensatzes umfassen Verkalkungsgrad-Labels, wobei die Verkalkungsgrad-Labels mindestens ein leichtes Label, ein mittleres Label und ein schweres Label umfassen;
die Herzcharakteristik bezieht sich auf eine Herzphase und die Klassen des Klassensatzes umfassen Prozentintervalle oder Prozent-Labels; der Untersuchungsbereich bezieht sich auf einen linken Vorhofanhang und die Herzcharakteristik bezieht sich auf eine Füllung des linken Vorhofanhangs, wobei die Klassen des Klassensatzes Prozentintervalle oder Prozent-Labels umfassen.

13. Verfahren zum Trainieren eines Datenverarbeitungsmodells (23, 23', 23") für maschinelles Lernen, wobei das Datenverarbeitungsmodell für maschinelles Lernen zum Durchführen eines Verfahrens gemäß einem oder mehreren der vorhergehenden Ansprüche konfiguriert ist, um ein Bildmerkmal (13) zu klassifizieren, um aus Herzbilddaten (21) eine Herzcharakteristik eines Patienten zu bestimmen, indem eine Wahrscheinlichkeit ausgewertet wird, dass das Bildmerkmal mit jeder Klasse (25, 26) einer vorbestimmten Vielzahl von Klassen übereinstimmt, die einen Klassensatz bilden, um das Bildmerkmal zu charakterisieren, wobei das Verfahren zum Trainieren Folgendes umfasst:
a) Empfangen eines Trainingsdatensatzes, der Trainingsdaten umfasst, durch das Datenverarbeitungsmodell für maschinelles Lernen, wobei die Trainingsdaten Herzbilddaten von mindestens einem Teil eines Herzbildes und optional Daten enthalten, die auf einen Untersuchungsbereich hinweisen, der das Bildmerkmal enthält;
b) Erhalten einer Vielzahl von Klassen, die einen Klassensatz bilden, durch das Datenverarbeitungsmodell für maschinelles Lernen, wobei die Klassen zusammen Statusklassen der Herzcharakteristik beschreiben;
c) Empfangen eines Grundwahrheitsdatensatzes, der Grundwahrheitsdaten umfasst, die auf eine Grundwahrheitsklasse hinweisen, die mit dem Bildmerkmal übereinstimmt, durch das Datenverarbeitungsmodell für maschinelles Lernen;
d) Trainieren des Datenverarbeitungsmodells für maschinelles Lernen auf der Grundlage der in Schritt a) erhaltenen Trainingsdaten, der in Schritt b) erhaltenen Klassen des Klassensatzes und der in Schritt c) erhaltenen Grundwahrheitsdaten, um das Datenverarbeitungsmodell für maschinelles Lernen in die Lage zu versetzen, nach Abschluss der Trainingsperiode den Schritt des Bereitstellens von Wahrscheinlichkeitsdaten durchzuführen, wobei die Wahrscheinlichkeitsdaten für jede Klasse des Klassensatzes eine Wahrscheinlichkeit umfassen, dass das Bildmerkmal mit der jeweiligen Klasse übereinstimmt.

14. Elektronisches Bildverarbeitungssystem (82) zur Verwendung in einem Verfahren nach einem oder mehreren der Ansprüche 1 bis 12, wobei das System ein trainiertes Datenverarbeitungsmodell für maschinelles Lernen (23, 23', 23") nach Anspruch 13 umfasst, um ein Bildmerkmal (13) zu klassifizieren, um aus Herzbilddaten eine Herzcharakteristik eines Patienten zu bestimmen, indem eine Wahrscheinlichkeit ausgewertet wird, dass das Bildmerkmal übereinstimmt mit jeder Klasse (25, 26) einer vorbestimmten Vielzahl von Klassen, die einen Klassensatz bilden, um das Bildmerkmal zu charakterisieren, wobei die Herzbilddaten mindestens einen Untersuchungsbereich umfassen, der das Bildmerkmal enthält, wobei das System ferner einen oder mehrere Prozessoren und einen Speicher umfasst, der das trainierte Datenverarbeitungsmodell für maschinelles Lernen und einen oder mehrere Befehle speichert, die, wenn sie in den Speicher geladen werden, den einen oder die mehreren Prozessoren in die Lage versetzen, die folgenden Schritte auszuführen:
Empfangen der Herzbilddaten durch das trainierte Datenverarbeitungsmodell für maschinelles Lernen;
Erhalten von Daten, die auf den Untersuchungsbereich hinweisen, durch das trainierte Datenverarbeitungsmodell für maschinelles Lernen;
Sammeln von Wahrscheinlichkeitsdaten aus dem trainierten Datenverarbeitungsmodell für maschinelles Lernen, wobei die Wahrscheinlichkeitsdaten für jede Klasse des Klassensatzes eine Wahrscheinlichkeit umfassen, dass das Bildmerkmal mit der jeweiligen Klasse übereinstimmt;
Auswerten der Wahrscheinlichkeitsdaten für die Klassen des Klassensatzes durch eine Steuerung und Identifizieren einer oder mehrerer Kandidatenklassen mit einer signifikanten Wahrscheinlichkeit der Übereinstimmung mit dem Bildmerkmal;
Bestimmen eines Gewissheitsgrads auf Grundlage der Wahrscheinlichkeitsdaten für jede der identifizierten Kandidatenklassen auf Grundlage eines Vergleichs der in dem Auswertungsschritt erhaltenen Wahrscheinlichkeiten, wobei der Gewissheitsgrad die Gewissheit angibt, dass das Bildmerkmal mit einer bestimmten Kandidatenklasse übereinstimmt; und
in Abhängigkeit von dem Schritt der Bestimmung mindestens einen der folgenden Schritte ausführt:
wenn die Anzahl der Kandidatenklassen gleich eins ist und der Gewissheitsgrad einen Schwellenwert überschreitet:
Assoziieren der einen Kandidatenklasse mit dem Bildmerkmal; oder
wenn die Anzahl der Kandidatenklassen gleich eins ist und der Gewissheitsgrad kleiner als ein Schwellenwert ist:
Anforderung einer Benutzereingabe, die eine Bestätigung oder Nichtbestätigung der Übereinstimmung der einen Kandidatenklasse mit dem Bildmerkmal anzeigen; oder
wenn die Anzahl der Kandidatenklassen größer als eins ist:
Anforderung einer Benutzereingabe für jede der einen oder mehreren Kandidatenklassen, die eine Bestätigung oder Nichtbestätigung der Übereinstimmung der jeweiligen Kandidatenklasse mit dem Bildmerkmal anzeigen.

## Revendications

1. Procédé, dans un système électronique de traitement d'image (82), de classification d'une caractéristique d'image (13) afin de déterminer, à partir de données d'image cardiaque (21), une caractéristique cardiaque d'un patient en utilisant un modèle entraîné de traitement de données d'apprentissage automatique (23, 23', 23"), dans lequel le modèle entraîné de traitement de données d'apprentissage automatique est entraîné à évaluer une probabilité que la caractéristique d'image corresponde à chaque classe d'une pluralité prédéterminée de classes formant un ensemble de classes de manière à caractériser la caractéristique d'image, les données d'image cardiaque comprenant au moins une région d'intérêt incluant la caractéristique d'image, dans lequel le procédé comprend le fait de :
recevoir (52), par le biais du modèle entraîné de traitement de données d'apprentissage automatique, les données d'image cardiaque ;
obtenir (54), par le biais du modèle entraîné de traitement de données d'apprentissage automatique, des données indicatives de la région d'intérêt ;
collecter (56), à partir du modèle entraîné de traitement de données d'apprentissage automatique, des données de probabilité, les données de probabilité comprenant, pour chaque classe (25, 26) de l'ensemble de classes, une probabilité que la caractéristique d'image corresponde à la classe respective ;
dans lequel le procédé comprend en outre les étapes comprenant le fait de :
évaluer (58), par le biais d'un contrôleur, les données de probabilité pour les classes de l'ensemble de classes, et identifier une ou plusieurs classes candidates ayant une probabilité significative de correspondre à la caractéristique d'image ;
déterminer (60) un niveau de certitude (30) sur la base des données de probabilité pour chacune des classes candidates identifiées en fonction d'une comparaison des probabilités obtenues à l'étape d'évaluation, dans lequel le niveau de certitude est indicatif de la certitude que la caractéristique d'image corresponde à une classe candidate particulière ; et
en fonction de l'étape de détermination, effectuer au moins une étape parmi :
lorsque le nombre de classes candidates est égal à un, et que le niveau de certitude dépasse un niveau de seuil : associer (68) ladite une classe candidate à la caractéristique d'image ; ou
lorsque le nombre de classes candidates est égal à un, et que le niveau de certitude est inférieur à un niveau de seuil : demander (70) une entrée utilisateur (34) indicative d'une confirmation ou d'une infirmation que ladite une classe candidate correspond à la caractéristique d'image ; ou
lorsque le nombre de classes candidates est supérieur à un : demander (72), pour chacune de la ou des classes candidates, une entrée utilisateur indicative d'une confirmation ou d'une infirmation que la classe candidate respective correspond à la caractéristique d'image.

2. Procédé selon la revendication 1, comprenant en outre le fait de fournir une sortie à un utilisateur (35) du système, dans laquelle la sortie comprend au moins un élément d'un groupe comprenant : des données indicatives de la ou des classes candidates ; des données d'une association entre une classe candidate et la caractéristique d'image ; le niveau de certitude ; ou les données de probabilité.

3. Procédé selon la revendication 1 ou 2, dans lequel, pour fournir les données de probabilité, le modèle entraîné de traitement de données d'apprentissage automatique comprend une couche gaussienne (10, 10') afin de déterminer, pour chaque classe de l'ensemble de classes, ladite probabilité que la caractéristique d'image corresponde à la classe respective.

4. Procédé selon l'une quelconque ou plusieurs des revendications précédentes, dans lequel le modèle entraîné de traitement de données d'apprentissage automatique est un réseau de neurones convolutif (1, 8) incluant une ou plusieurs couches intermédiaires (7, 7'), et dans lequel l'étape de collecte comprend en outre le fait de :
obtenir, à partir d'au moins une des couches intermédiaires, un vecteur de pondération de M poids et des données intermédiaires de M canaux, dans lequel M comprend un nombre naturel supérieur à zéro ;
pondérer les données intermédiaires au moyen des poids, ce qui donne des données intermédiaires pondérées ; et
échantillonner les données intermédiaires pondérées, ce qui donne des données indicatives de région, les données indicatives de région étant indicatives d'une ou de plusieurs régions d'importance de l'image cardiaque ayant une pondération supérieure à la moyenne dans les données intermédiaires pondérées.

5. Procédé selon l'une quelconque ou plusieurs des revendications 1 à 3, dans lequel le modèle entraîné de traitement de données d'apprentissage automatique comprend une couche d'entrée (4) destinée à recevoir au moins les données d'image cardiaque, et une couche de sortie (5) destinée à fournir des données de sortie, les données de sortie incluant au moins l'un parmi : des données indicatives de la ou des classes candidates, une association entre une classe candidate et la caractéristique d'image, le niveau de certitude, ou les données de probabilité ;
dans lequel le procédé comprend en outre les étapes comprenant le fait de :
modifier, par le biais du contrôleur, une ou plusieurs parties des données d'image cardiaque ;
évaluer, par le biais du modèle entraîné de traitement de données d'apprentissage automatique, une modification dans les données de sortie en réponse à l'étape de modification, dans laquelle la modification se rapporte à au moins l'un parmi les classes candidates, l'association, le niveau de certitude, ou les données de probabilité ; et
identifier, sur la base de l'étape d'évaluation, une ou plusieurs régions d'importance dans les données d'image cardiaque ; et
fournir des données indicatives de région, dans lesquelles les données indicatives de région sont indicatives de la ou des régions d'importance.

6. Procédé selon la revendication 4 ou 5, comprenant en outre le fait de fournir une sortie à un utilisateur du système, dans laquelle la sortie comprend les données indicatives de région.

7. Procédé selon l'une quelconque ou plusieurs des revendications 4 à 6, dans lequel l'étape d'échantillonnage comprend au moins l'un parmi : un suréchantillonnage, tel qu'un suréchantillonnage bilinéaire ou une interpolation ; et un sous-échantillonnage, tel qu'une décimation.

8. Procédé selon une ou plusieurs des revendications précédentes, dans lequel l'étape d'obtention des données indicatives de la région d'intérêt comprend au moins l'une des étapes comprenant le fait de :
obtenir une entrée utilisateur indicative de la région d'intérêt ; ou
effectuer une opération de filtrage de prétraitement pour établir les données indicatives de la région d'intérêt ; ou
effectuer, en utilisant le ou un autre modèle entraîné de traitement de données d'apprentissage automatique, une étape de reconnaissance d'image de prétraitement pour établir les données indicatives de la région d'intérêt.

9. Procédé selon l'une quelconque ou plusieurs des revendications précédentes, dans lequel les données d'image cardiaque comprennent l'une ou plusieurs parmi : des données de tomodensitométrie, des données de tomographie par émission de positons, des données d'imagerie par résonance magnétique, des données d'imagerie ultrasonore telles que des données d'imagerie échocardiographique, des données d'imagerie radiographique, des données d'imagerie angiographique.

10. Procédé selon l'une quelconque ou plusieurs des revendications précédentes, dans lequel la région d'intérêt est liée à un atrium du patient et les classes de l'ensemble de classes comprennent des étiquettes de morphologie de valve aortique.

11. Procédé selon la revendication 10, dans lequel les étiquettes de morphologie de valve aortique comprennent au moins l'une ou plusieurs parmi : une étiquette de valve aortique bicuspide de type 0, une étiquette de valve aortique bicuspide de type 1, une étiquette de valve aortique tricuspide, une étiquette de valve aortique quadricuspide, une étiquette de valve aortique pentacuspide, une étiquette de valve aortique unicuspide, ou une étiquette de valve aortique à n cuspides.

12. Procédé selon l'une quelconque ou plusieurs des revendications 1 à 9, dans lequel au moins l'un de ce qui suit s'applique :
la région d'intérêt est liée à un appendice auriculaire gauche et les classes de l'ensemble de classes comprennent des étiquettes de morphologie d'appendice auriculaire gauche, telles qu'une étiquette chou-fleur ou une étiquette aile de poulet ; ou
la région d'intérêt est liée à une valve mitrale du patient et les classes de l'ensemble de classes comprennent des étiquettes de degré de calcification, des lesquelles les étiquettes de degré de calcification comprennent au moins une étiquette légère, une étiquette modérée et une étiquette sévère ; ou
la région d'intérêt est liée à une racine aortique du patient et les classes de l'ensemble de classes comprennent des étiquettes de degré de calcification, dans lesquelles les étiquettes de degré de calcification comprennent au moins une étiquette légère, une étiquette modérée et une étiquette sévère ; ou
la caractéristique cardiaque se rapporte à une phase cardiaque et les classes de l'ensemble de classes comprennent des intervalles de pourcentage ou des étiquettes de pourcentage ;
la région d'intérêt est liée à un appendice auriculaire gauche et la caractéristique cardiaque se rapporte à un remplissage d'appendice auriculaire gauche, dans lequel les classes de l'ensemble de classes comprennent des intervalles de pourcentage ou des étiquettes de pourcentage.

13. Procédé d'entraînement d'un modèle de traitement de données d'apprentissage automatique (23, 23', 23"), dans lequel le modèle de traitement de données d'apprentissage automatique est configuré pour mettre en œuvre un procédé selon l'une quelconque ou plusieurs des revendications précédentes, pour classifier une caractéristique d'image (13) afin de déterminer, à partir de données d'image cardiaque (21), une caractéristique cardiaque d'un patient en évaluant une probabilité que la caractéristique d'image corresponde à chaque classe (25, 26) d'une pluralité prédéterminée de classes formant un ensemble de classes de manière à caractériser la caractéristique d'image, le procédé d'entraînement comprenant le fait de :
a) recevoir, par le biais du modèle de traitement de données d'apprentissage automatique, un ensemble de données d'entraînement comprenant des données d'entraînement, les données d'entraînement incluant des données d'image cardiaque d'au moins une partie d'une image cardiaque, et éventuellement des données indicatives d'une région d'intérêt qui inclut la caractéristique d'image ;
b) obtenir, par le biais du modèle de traitement de données d'apprentissage automatique, une pluralité de classes formant un ensemble de classes, dans lequel les classes décrivent ensemble des classes d'état de la caractéristique cardiaque ;
c) recevoir, par le biais du modèle de traitement de données d'apprentissage automatique, un ensemble de données de vérité terrain comprenant des données de vérité terrain indicatives d'une classe de vérité terrain qui correspond à la caractéristique d'image ;
d) entraîner le modèle de traitement de données d'apprentissage automatique sur la base des données d'entraînement reçues à l'étape a), des classes de l'ensemble de classes obtenues à l'étape b), et des données de vérité terrain reçues à l'étape c), pour permettre au modèle de traitement de données d'apprentissage automatique, après achèvement de la période d'entraînement, d'effectuer l'étape de fourniture de données de probabilité, les données de probabilité comprenant, pour chaque classe de l'ensemble de classes, une probabilité que la caractéristique d'image corresponde à la classe respective.

14. Système électronique de traitement d'image (82) destiné à être utilisé dans un procédé selon l'une quelconque ou plusieurs des revendications 1 à 12, le système comprenant un modèle entraîné de traitement de données d'apprentissage automatique (23, 23', 23") selon la revendication 13, pour classifier une caractéristique d'image (13) afin de déterminer, à partir de données d'image cardiaque, une caractéristique cardiaque d'un patient en évaluant une probabilité que la caractéristique d'image corresponde à chaque classe (25, 26) d'une pluralité prédéterminée de classes formant un ensemble de classes de manière à caractériser la caractéristique d'image, les données d'image cardiaque comprenant au moins une région d'intérêt incluant la caractéristique d'image, dans lequel le système comprend en outre un ou plusieurs processeurs et une mémoire stockant le modèle entraîné de traitement de données d'apprentissage automatique et une ou plusieurs instructions qui, lorsqu'elles sont chargées dans la mémoire, permettent aux un ou plusieurs processeurs d'exécuter les étapes comprenant le fait de :
recevoir, par le biais du modèle entraîné de traitement de données d'apprentissage automatique, les données d'image cardiaque ;
obtenir, par le biais du modèle entraîné de traitement de données d'apprentissage automatique, des données indicatives de la région d'intérêt ;
collecter, à partir du modèle entraîné de traitement de données d'apprentissage automatique, des données de probabilité, les données de probabilité comprenant, pour chaque classe de l'ensemble de classes, une probabilité que la caractéristique d'image corresponde à la classe respective ;
dans lequel le procédé comprend en outre les étapes comprenant le fait de :
évaluer, par le biais d'un contrôleur, les données de probabilité pour les classes de l'ensemble de classes, et identifier une ou plusieurs classes candidates ayant une probabilité significative de correspondre à la caractéristique d'image ;
déterminer un niveau de certitude sur la base des données de probabilité pour chacune des classes candidates identifiées en fonction d'une comparaison des probabilités obtenues à l'étape d'évaluation, dans lequel le niveau de certitude est indicatif de la certitude que la caractéristique d'image corresponde à une classe candidate particulière ; et
en fonction de l'étape de détermination, exécuter au moins une étape parmi :
lorsque le nombre de classes candidates est égal à un, et que le niveau de certitude dépasse un niveau de seuil : associer ladite une classe candidate à la caractéristique d'image ; ou
lorsque le nombre de classes candidates est égal à un, et que le niveau de certitude est inférieur à un niveau de seuil : demander une entrée utilisateur indicative d'une confirmation ou d'une infirmation que ladite une classe candidate correspond à la caractéristique d'image ; ou
lorsque le nombre de classes candidates est supérieur à un : demander, pour chacune de la ou des classes candidates, une entrée utilisateur indicative d'une confirmation ou d'une infirmation que la classe candidate respective correspond à la caractéristique d'image.
